# NEUE EUROPÄISCHE PATENTSCHRIFT

(11) **EP 2 483 190 B3**
(45) Veröffentlichungstag dieser Patentschrift: **14.08.2024**
(45) Hinweis auf die Patenterteilung: 16.04.2014
(21) Anmeldenummer: 10763581.5
(22) Anmeldetag: 01.10.2010
(51) Int. Cl.: B65H 63/06, D01H 13/22, G01N 33/36

(54) **VERFAHREN ZUM FESTLEGEN EINER REINIGUNGSGRENZE AUF EINER GARNREINIGUNGSANLAGE**
METHOD FOR ESTABLISHING A CLEARING LIMIT OF A YARN CLEARING SYSTEM
PROCÉDÉ D'ÉTABLISSEMENT D'UNE LIMITE DE NETTOYAGE SUR UNE INSTALLATION DE NETTOYAGE DE FIL

(30) Priorität: 02.10.2009 CH 15252009
(43) Veröffentlichungstag der Anmeldung: 08.08.2012
(73) Patentinhaber: Uster Technologies AG, 8610 Uster (CH)
(72) Erfinder: STORZ, Rafael, CH-8280 Kreuzlingen (CH); SCHMID, Peter, CH-8050 Zürich (CH); PIRANI, Peter, CH-8624 Grüt (CH); NARAYANAN, Sivakumar, CH-8610 Uster (CH); KELLER, Beat, CH-8046 Zürich (CH); GEHRIG, Stefan, CH-8610 Uster (CH)
(86) Internationale Anmeldenummer: PCT/CH2010/000238
(87) Internationale Veröffentlichungsnummer: WO 2011/038524

(56) Entgegenhaltungen:
- EP-A2- 0 415 222
- EP-A2- 0 415 222
- US-A- 5 178 008
- US-A- 5 178 008
- US-B1- 6 374 152
- US-B1- 6 374 152

## Beschreibung

### FACHGEBIET

Die vorliegende Erfindung liegt auf dem Gebiet der textilen Qualitätskontrolle. Sie betrifft Verfahren zum Festlegen einer Reinigungsgrenze auf einer elektronischen Reinigungsanlage für ein längliches textiles Prüfgut wie Garn, gemäss den unabhängigen Patentansprüchen. Die Verfahren kommen vorzugsweise bei Garnreinigern auf Spinn- oder Spulmaschinen für Garn zum Einsatz.

### STAND DER TECHNIK

Zur Sicherung der Garnqualität werden an Spinn- oder Spulmaschinen so genannte Garnreiniger eingesetzt. Eine derartige Vorrichtung ist z. B. aus der EP-1'249'422 A2 bekannt. Sie beinhaltet einen Messkopf mit mindestens einem Sensor, der das bewegte Garn abtastet. Häufig verwendete Sensorprinzipien sind das kapazitive (siehe z. B. EP-0'924'513 A1) oder das optische (siehe z. B. WO-93/13407 A1). Ziel der Abtastung ist es, Fehlstellen wie Dickstellen, Dünnstellen oder Fremdstoffe im Garn zu detektieren. Das Ausgangssignal des Sensors wird laufend mit vorgegebenen Bewertungskriterien bewertet. Die Bewertungskriterien werden üblicherweise in Form einer Reinigungsgrenze in einem zweidimensionalen Klassierfeld oder Ereignisfeld vorgegeben, welches von der Länge des Ereignisses einerseits und von einer Amplitude des Ereignisses, z. B. einer Abweichung der Garnmasse von einem Sollwert, andererseits aufgespannt wird. Ereignisse unterhalb der Reinigungsgrenze werden toleriert, Ereignisse oberhalb der Reinigungsgrenze werden aus dem Garn entfernt oder zumindest als Fehlstellen registriert.

Die EP-1'295'835 A2 offenbart ein Verfahren zum Einstellen einer Reinigungsgrenze bei einem elektronischen Garnreiniger. Die möglichen Garnfehler sind in einem Sortierschema, sortiert nach Fehlerwert und Fehlerlänge, angeordnet. Die Reinigungsgrenze wird mittels einer Kurve ausgewählt und am Garnreiniger eingestellt. Zu diesem Zweck wird ein Kurvenverlauf für die die Reinigungsgrenze darstellende Kurve ausgewählt, wobei der Kurvenverlauf an sich beliebig aber definiert ist. Die Reinigungskurve wird durch Verschieben genau eines Punktes im Sortierschema verschoben, bis ihre Lage der verschiebenden Bedienungsperson geeignet scheint.

Die EP-0'415'222 A2 stellt sich die Aufgabe, eine optimale Einstellung der Ansprechgrenzen bezüglich der Garnfeinheit anhand der tatsächlichen Produktionsgegebenheiten zu ermöglichen. Zu diesem Zweck wird die längenbezogene Anzahl der zulässigen Fehleralarme eingegeben. Während des Reinigungsprozesses werden die Messwerte der Garnfeinheit laufend registriert, und es wird ihre Verteilung bestimmt. Aus dieser Verteilung der Messwerte und aus der vorgegebenen zulässigen Alarmhäufigkeit werden anhand statistischer Gesetzmässigkeiten die Ansprechgrenzen automatisch festgelegt.

Gemäss der EP-0'439'767 A2 und der US-5,178,008 A wird die Reinigungsgrenze festgelegt, indem die Garnfehler ermittelt, klassiert und gezählt werden. Dabei werden die Einstellparameter Bezugslänge (die im Wesentlichen der Fehlerlänge entspricht) und Empfindlichkeit (die im Wesentlichen dem Fehlerwert entspricht) in Relation zur Anzahl der entsprechenden Reinigerschnitte gesetzt. Es wird ein Reinigungsprofil erstellt, welches den funktionalen Zusammenhang zwischen der Menge aller möglichen Kombinationen der genannten Einstellparameter und den Reinigereingriffen darstellt. Das Reinigungsprofil wird in einem dreidimensionalen rechtwinkligen Koordinatensystem als gewölbte Fläche dargestellt. Dem Reinigungsprofil ist für beliebige Kombinationen der Einstellparameter die zu erwartende Anzahl von Reinigereingriffen entnehmbar. Ferner wird eine "virtuelle Garnreinigung" beschrieben, bei welcher eine virtuelle Reinigungsgrenze festgelegt wird, die unterhalb der aktuellen Reinigungsgrenze liegt. Die virtuelle Reinigungsgrenze löst keine Garnschnitte aus; stattdessen dient sie nur der Zählung derjenigen Garnereignisse, die herausgeschnitten worden wären, wenn die virtuelle Reinigungsgrenze angewendet worden wäre.

Aus der US-6,374,152 B1 ist ein Verfahren zum Reinigen von Garnen bekannt, in dem die Reinigungsgrenze aufgrund der Dichte der Garnfehler selbsttätig berechnet und so optimal eingestellt wird. Zu diesem Zweck wird ein genügend langer Abschnitt des Garns vorgängig ausgemessen. Die gemessenen Ereignisse werden im bekannten zweidimensionalen Klassierfeld als Punkte oder Kreuze dargestellt. Aus den Messresultaten wird die Garnfehlerdichte berechnet und im Klassierfeld als Relief dargestellt. Die Reinigungsgrenze wird aufgrund einer vorgegebenen zulässigen Garnfehlerdichte selbsttätig festgelegt. Sie kann während des Kontrollvorgangs laufend automatisch nachgeregelt werden. Die Messungen erfolgen in den Garnreinigermessköpfen an den einzelnen Spinn- oder Spulstellen, während die Berechnungen und Regelungen in einer zentralen Recheneinheit stattfinden, mit der eine Vielzahl von Garnreinigermessköpfen verbunden ist.

Ein Nachteil der aus dem Stand der Technik bekannten Verfahren ist darin zu sehen, dass sie sich einseitig auf die zu erzielende Garnqualität konzentrieren, dabei aber die Produktionseffizienz zu wenig berücksichtigen. Auch sind sie teilweise wenig benutzerfreundlich.

### DARSTELLUNG DER ERFINDUNG

Der vorliegenden Erfindung liegt die Aufgabe zugrunde, Verfahren zum Festlegen einer Reinigungsgrenze auf einer elektronischen Reinigungsanlage für ein längliches textiles Prüfgut anzugeben, die eine möglichst effiziente Ausnutzung der textilen Produktionsmaschine unter Berücksichtigung des zu reinigenden Prüfguts ermöglichen. Die Verfahren sollen benutzerfreundlich und einfach auszuführen sein. Die Bedienungsperson soll bei der Festlegung der Reinigungsgrenze von der Reinigungsanlage unterstützt werden, aber dennoch die Möglichkeit haben, bei Bedarf die Festlegung zu beeinflussen.

Diese und andere Aufgaben werden durch die erfindungsgemässen Verfahren gemäss den unabhängigen Patentansprüchen gelöst. Vorteilhafte Ausführungsformen sind in den abhängigen Patentansprüchen angegeben.

Gemäss der Erfindung wird zuerst eine statistische Repräsentanz des Prüfgutes mittels Messungen am Prüfgut ermittelt. Aufgrund der statistischen Repräsentanz wird eine Reinigungsgrenze berechnet und zur Anwendung vorgeschlagen, wobei eine mit dieser Reinigungsgrenze zu erwartende längenbezogene Anzahl unzulässiger Ereignisse berechnet und ausgegeben wird. Eine Bedienungsperson kann zur zu erwartenden Anzahl unzulässiger Ereignisse Stellung nehmen, wonach die Reinigungsgrenze gemäss der Stellungnahme automatisch festgelegt wird.

Durch ihre Stellungnahme kann die Bedienungsperson bestimmen, ob die vorgeschlagene Reinigungsgrenze für die gewünschte Anwendung ausreichend ist, ob sie verschärft oder entschärft werden soll. "Verschärfen" bedeutet hier, dass mehr Garnereignisse ausgereinigt werden sollen, "Entschärfen" bedeutet, dass weniger Garnereignisse ausgereinigt werden sollen. Das System nimmt daraufhin Korrekturen an der Reinigungsgrenze vor, die das gewünschte Verhalten zur Folge haben.

Die Reinigungsgrenze kann z. B. so berechnet werden, dass sie im Wesentlichen einer konstanten Dichte von Ereignissen folgt. Bei der Berechnung der Reinigungsgrenze kann eine Einteilung der möglichen Prüfgüter in verschiedene Anwendungsgebiete berücksichtigt werden. Es kann also durch einprogrammiertes oder gelerntes Systemwissen unterschieden werden, an welchen Stellen die Reinigungsgrenze optimiert werden soll, damit ein möglichst optimales Reinigungsergebnis erzielt wird. Um diese Einstellungen für die Bedienungsperson möglichst einfach zu machen, wird ihr die Möglichkeit angeboten, durch einfachen Mausklick oder Tastendruck um einen Inkrementwert die Anzahl der zu erwartenden Reinigungsschnitte pro Längeneinheit zu erhöhen oder zu erniedrigen.

In einer bevorzugten Ausführungsform des erfindungsgemässen Verfahrens wird der Bedienungsperson nach jeder Neuberechnung der Reinigungsgrenze und der längenbezogenen Anzahl unzulässiger Ereignisse Gelegenheit zur Stellungnahme geboten. Dadurch kann die Bedienungsperson die Reinigungsgrenze so lange ändern, bis die berechnete längenbezogene Anzahl unzulässiger Ereignisse einen gewünschten Wert annimmt und von der Bedienungsperson bestätigt wird. Dabei kann immer auf die anfänglich ermittelte statistische Repräsentanz zurückgegriffen werden; es braucht also keine neue Messung durchgeführt zu werden. Diese Ausführungsform funktioniert also rekursiv, indem aufgrund einer einmal ermittelten statistischen Repräsentanz immer wieder eine neue Reinigungsgrenze und die dazugehörige längenbezogene Anzahl unzulässiger Ereignisse berechnet werden, bis die Bedienungsperson die aktuelle Reinigungsgrenze bestätigt.

Die statistische Repräsentanz ergibt sich aus einer Messung und Erfassung einer genügend grossen Anzahl von Ereignissen am zu untersuchenden Prüfgut. Je grösser die Anzahl erfasster Ereignisse, desto sicherer und aussagekräftiger ist die vorausgesagte längenbezogene Anzahl unzulässiger Ereignisse, weil es sich bei der Voraussage im Wesentlichen um eine Extrapolation handelt. Theoretische Überlegungen und Experimente zeigen, dass in den meisten Fällen die Ausmessung von 100 km Garn genügt, um eine befriedigende statistische Repräsentanz zu ermitteln. Diese 100 km brauchen nicht auf einer einzigen Spulstelle umgespult zu werden, sondern können aus mehreren Spulstellen zusammengesetzt werden, auf denen derselbe Garntyp umgespult wird. In Ausnahmefällen können kürzere Einmesslängen, z. B. 50 km, genügen, oder grössere Einmesslängen, z. B. 500 km, nötig sein.

Die erfindungsgemässen Verfahren dienen zum Festlegen einer Reinigungsgrenze auf einer elektronischen Reinigungsanlage für ein längliches textiles Prüfgut wie Garn. Gemäss einem ersten Aspekt werden Messwerte mindestens einer Eigenschaft des entlang seiner Längsrichtung bewegten Prüfgutes auf einer bestimmten Prüfgutlänge erfasst. Aus den Messwerten werden Ereignisse im Prüfgut identifiziert. Eine statistische Repräsentanz des Prüfgutes wird aus den Ereignissen ermittelt. Aufgrund der statistischen Repräsentanz wird dem Prüfgut automatisch ein bestimmter Prüfguttyp zugeordnet. Eine Reinigungsgrenze wird als Kriterium für die Zulässigkeit bzw. Unzulässigkeit der Ereignisse aufgrund der statistischen Repräsentanz berechnet, wobei der zugeordnete Prüfguttyp die Berechnung der Reinigungsgrenze beeinflusst. Eine längenbezogene Anzahl unzulässiger Ereignisse wird aus der ermittelten statistischen Repräsentanz und der Reinigungsgrenze berechnet. Die längenbezogene Anzahl unzulässiger Ereignisse wird auf einer Ausgabeeinheit ausgegeben. Eine Bedienungsperson wird aufgefordert, eine Stellungnahme zur ausgegebenen längenbezogenen Anzahl unzulässiger Ereignisse mittels einer Eingabeeinheit einzugeben. Die Reinigungsgrenze wird dann gemäss der eingegebenen Stellungnahme automatisch festgelegt.

Ein zweiter Aspekt der Erfindung betrifft ebenfalls ein Verfahren zum Festlegen einer Reinigungsgrenze auf einer elektronischen Reinigungsanlage für ein längliches textiles Prüfgut wie Garn. Messwerte mindestens einer Eigenschaft des entlang seiner Längsrichtung bewegten Prüfgutes werden auf einer bestimmten Prüfgutlänge erfasst. Aus den Messwerten werden Ereignisse im Prüfgut identifiziert. Eine statistische Repräsentanz des Prüfgutes wird aus den Ereignissen ermittel, Eine Reinigungsgrenze wird als Kriterium für die Zulässigkeit bzw. Unzulässigkeit der Ereignisse aufgrund der statistischen Repräsentanz berechnet, wobei eine für die Zukunft vorgesehene Verwendung des Prüfgutes die Berechnung der Reinigungsgrenze beeinflusst. Eine längenbezogene Anzahl unzulässiger Ereignisse wird aus der ermittelten statistischen Repräsentanz und der Reinigungsgrenze berechnet. Die längenbezogene Anzahl unzulässiger Ereignisse wird auf einer Ausgabeeinheit ausgegeben. Eine Bedienungsperson wird aufgefordert, eine Stellungnahme zur ausgegebenen längenbezogenen Anzahl unzulässiger Ereignisse mittels einer Eingabeeinheit einzugeben. Die Reinigungsgrenze wird gemäss der eingegebenen Stellungnahme automatisch festgelegt.

Bei komplexeren Anforderungen wie der Grundreinigung (NSLT-Fehler) und der gleichzeitigen Ausreinigung von Fremdstofffehlern ist es vorteilhaft, eine Separation der einzelnen Ausreinigungsanforderungen vorzunehmen: Die Grundreinigung erhält einen ersten Teil der zulässigen Schnittzahl, die zweite Ausreinigungsanforderung erhält den verbleibenden, zum ersten Teil komplementären Teil der noch zulässigen Schnittzahl. Dementsprechend betrifft auch ein dritter Aspekt der Erfindung ein Verfahren zum Festlegen einer Reinigungsgrenze auf einer elektronischen Reinigungsanlage für ein längliches textiles Prüfgut wie Garn. Messwerte mehrerer Eigenschaften des entlang seiner Längsrichtung bewegten Prüfgutes werden auf einer bestimmten Prüfgutlänge erfasst. Aus den Messwerten werden Ereignisse im Prüfgut identifiziert. Für jede der Eigenschaften wird aus den Ereignissen eine statistische Repräsentanz des Prüfgutes ermittelt. Für jede der Eigenschaften wird eine Reinigungsgrenze als Kriterium für die Zulässigkeit bzw. Unzulässigkeit der Ereignisse berechnet. Für jede der Eigenschaften wird eine sich aus der ermittelten statistischen Repräsentanz und der Reinigungsgrenze ergebende längenbezogene Teilanzahl unzulässiger Ereignisse berechnet. Eine längenbezogene Gesamtanzahl unzulässiger Ereignisse wird als Summe der Teilanzahlen berechnet. Verhältnisse der Teilanzahlen werden, vorzugsweise automatisch, derart festgelegt, dass die Gesamtanzahl unzulässiger Ereignisse unverändert bleibt. Für jede der Eigenschaften wird die Reinigungsgrenze derart berechnet, dass sich aus der ermittelten statistischen Repräsentanz und der berechneten Reinigungsgrenze die festgelegte längenbezogene Teilanzahl unzulässiger Ereignisse ergibt. Die längenbezogene Gesamtanzahl unzulässiger Ereignisse wird auf einer Ausgabeeinheit ausgegeben. Eine Bedienungsperson wird aufgefordert, eine Stellungnahme zur ausgegebenen längenbezogenen Gesamtanzahl unzulässiger Ereignisse mittels einer Eingabeeinheit einzugeben. Die Reinigungsgrenze gemäss der eingegebenen Stellungnahme wird automatisch festgelegt.

Die Stellungnahme kann entweder eine Bestätigung, worauf die Reinigungsgrenze unverändert belassen wird, oder ein Änderungsbefehl sein. Im letzteren Fall wird eine neue Reinigungsgrenze gemäss dem Änderungsbefehl und aufgrund der statistischen Repräsentanz berechnet, und eine neue längenbezogene Anzahl unzulässiger Ereignisse wird aus der ermittelten statistischen Repräsentanz sowie der neuen Reinigungsgrenze berechnet und auf der Ausgabeeinheit ausgegeben.

Der Änderungsbefehl ist vorzugsweise entweder ein Befehl zur Erhöhung der längenbezogenen Anzahl unzulässiger Ereignisse oder ein Befehl zur Erniedrigung der längenbezogenen Anzahl unzulässiger Ereignisse. Die Erhöhung bzw. Erniedrigung der längenbezogenen Anzahl unzulässiger Ereignisse erfolgt z. B. um einen automatisch berechneten Inkrementwert, der im Wesentlichen proportional zur berechneten längenbezogenen Anzahl unzulässiger Ereignisse ist. Besonders bedienerfreundlich und einfach ist es, wenn die Bestätigung bzw. der Änderungsbefehl durch eine einzige Handlung, bspw. einen einzigen Knopfdruck, einen einzigen Mausklick oder einen einzige Berührung eines Sensorbildschirms, erfolgt. Nach einem Änderungsbefehl kann die Bedienungsperson wiederum aufgefordert werden, eine Stellungnahme zur ausgegebenen neuen längenbezogenen Anzahl unzulässiger Ereignisse mittels der Eingabeeinheit einzugeben, worauf die Reinigungsgrenze gemäss der eingegebenen Stellungnahme automatisch festgelegt wird.

Es ist von Vorteil, wenn die Reinigungsgrenze aufgrund der statistischen Repräsentanz derart berechnet wird, dass eine sich aus der ermittelten statistischen Repräsentanz und der Reinigungsgrenze ergebende längenbezogene Anzahl unzulässiger Ereignisse pro 100 km Prüfgutlänge zwischen 1 und 200, vorzugsweise zwischen 10 und 100, liegt.

Es kann vorteilhaft sein, zusätzlich zur besagten Reinigungsgrenze eine Spleissreinigungsgrenze als Kriterium für zulässige bzw. unzulässige Spleisse im Prüfgut festzulegen. Die besagte Reinigungsgrenze wird dann so festgelegt, dass sie immer oberhalb der Spleissreinigungsgrenze liegt.

Die festgelegte Reinigungsgrenze kann automatisch auf mindestens ein vorgegebenes Kriterium hin überprüft werden. Bei Nichterfüllung des Kriteriums wird eine Warnung ausgegeben.

Die mindestens eine Eigenschaft ist vorzugsweise eine Masse pro Längeneinheit, ein Durchmesser und/oder eine Zusammensetzung des Prüfgutes. Die Ereignisse können durch die Messwerte einerseits und durch eine Erstreckung von Messwerten in der Längsrichtung andererseits definiert werden. Es kann ein Ereignisfeld bereitgestellt werden, das einen Quadranten oder einen Teil eines Quadranten eines zweidimensionalen kartesischen Koordinatensystems beinhaltet, dessen Abszisse die Erstreckung der Messwerte in der Längsrichtung und dessen Ordinate eine Abweichung der Messwerte von einem Sollwert angibt. Aus den Messwerten und ihrer Erstreckung in der Längsrichtung werden Dichten von Ereignissen in dem Ereignisfeld ermittelt, und die Reinigungsgrenze folgt im Wesentlichen einer konstanten Ereignisdichte. Die Reinigungsgrenze kann aber auch von der Dichtelinie abweichen. Um solche Abweichungen einzugeben, können Stützpunkte vorgesehen sein, welche eine Bedienungsperson frei eingeben kann. Die Reinigungskurve wird dann so berechnet, dass sie durch die eingegebenen Stützpunkte verläuft und zwischen den Stützpunkten auf geeignete, an sich bekannte Weise interpoliert wird.

Die festgelegte Reinigungsgrenze wird vorzugsweise an mindestens einem Messkopf der elektronischen Reinigungsanlage eingestellt. Die Festlegung der Reinigungsgrenze erfolgt mit Vorteil in einer Steuereinheit der elektronischen Reinigungsanlage.

Besonders einfach und bedienungsfreundlich ist das Verfahren, wenn die Festlegung der Reinigungsgrenze automatisch erfolgt und durch eine einzige Handlung, bspw. einen einzigen Knopfdruck, einen einzigen Mausklick oder einen einzige Berührung eines Sensorbildschirms, ausgelöst wird.

Die erfindungsgemässe elektronische Reinigungsanlage für ein längliches textiles Prüfgut wie Garn, beinhaltet mindestens einen Messkopf zur Erfassung von Messwerten mindestens einer Eigenschaft des entlang seiner Längsrichtung bewegten Prüfgutes auf einer bestimmten Prüfgutlänge und eine mit dem Messkopf verbundene Steuereinheit. Die Steuereinheit ist zur Steuerung des oben beschriebenen erfindungsgemässen Verfahrens eingerichtet.

Vorzugsweise werden unterschiedliche vordefinierte Reinigungsgrenzen zur Verfügung gestellt. Eine Optimierung der Reinigungsgrenze könnte damit durch gezielte, von der Anwendung abhängende Parameter, vorgenommen werden.

Die erfindungsgemässe Vorrichtung kommt in einer Textilverarbeitungsmaschine, bspw. einer Spinn- oder Spulmaschine für Garn, zum Einsatz. Eine derartige Textilverarbeitungsmaschine weist typischerweise eine Vielzahl von Arbeitsstellen auf. Dementsprechend kann die erfindungsgemässe Vorrichtung eine Vielzahl von Messköpfen beinhalten, die sich an jeder Arbeitsstelle befinden. Die Messköpfe sind alle an der zentralen Steuereinheit angeschlossen, z. B. über einen seriellen Bus wie z. B. RS-485. Zwischen einem jeweiligen Messkopf und der Steuereinheit kann ein Schnittstellenwandler eingebaut sein. Die Steuereinheit ist vorzugsweise in der Textilverarbeitungsmaschine eingebaut. Dies hat den Vorteil, dass die Person, welche die Textilverarbeitungsmaschine bedient, die Einstellung der Reinigungsgrenze an ihrem Arbeitsplatz vornehmen kann, und dass kein zusätzlicher Rechner benötigt wird. Die erfindungsgemässe Vorrichtung kann an jeder Arbeitsstelle, in der Nähe eines jeden Messkopfes, eine Schneideinrichtung zur Entfernung von unzulässigen Ereignissen aus dem Garn beinhalten.

### AUFZÄHLUNG DER ZEICHNUNGEN

Nachfolgend wird die Erfindung am Beispiel einer Spulmaschine für Garn anhand von Zeichnungen detailliert erläutert.
- Figur 1: zeigt schematisch eine Spulmaschine mit einem Garnreinigersystem.
- Figur 2: zeigt ein Ereignisfeld mit einem Garnkörper und einer Reinigungsgrenze.
- Figur 3: zeigt Ereignisfelder mit Garnereignissen, einem Garnkörpern und Reinigungsgrenzen für drei verschiedene Garne.
- Figur 4: zeigt ein Flussdiagramm einer bevorzugten Ausführungsform des erfindungsgemässen Verfahrens.
- Figur 5: zeigt ein Bedienungsfeld einer erfindungsgemässen Vorrichtung.

### AUSFÜHRUNG DER ERFINDUNG

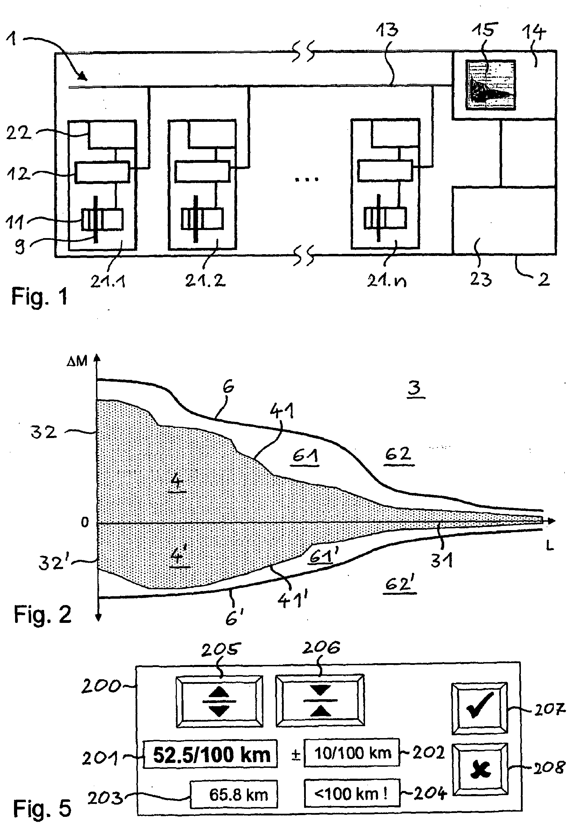

In **Figur 1** ist sehr schematisch eine Spulmaschine 2 mit mehreren Spulstellen 21.1, 21.2, ..., 21.n dargestellt. Eine erfindungsgemässe Garnreinigungsanlage 1 ist in die Spulmaschine 2 eingebaut. An jeder Spulstelle 21.1 wird während des Umspulvorgangs Garn 9 von einem Messkopf 11 der erfindungsgemässen Garnreinigungsanlage 1 überwacht. Der Messkopf 11 beinhaltet einen Sensor, mit dem eine Eigenschaft des Garns 9 gemessen wird, z. B. einen kapazitiven Sensor zur Messung einer dielektrischen Eigenschaft des Garns 9. Ferner beinhaltet der Messkopf 11 eine Auswerteeinheit, die dazu eingerichtet ist, aus den Messwerten einen Garnparameter, z. B. die Garnmasse pro Längeneinheit, zu ermitteln. Der Messkopf 11 ist über einen Schnittstellenwandler 12 mit einer zentralen Steuereinheit 14 der erfindungsgemässen Garnreinigungsanlage 1 verbunden. Über die Verbindung wird der Messkopf 11 von der Steuereinheit 14 eingestellt und gesteuert, und der Messkopf 11 übermittelt Daten wie die ermittelten Garnparameter an die Steuereinheit 14. Eine Verbindungsleitung 13 zwischen allen Schnittstellenwandlern 12 und der Steuereinheit 14 kann als serieller Bus wie z. B. RS-485 ausgebildet sein. Der Schnittstellenwandler 12 kann zusätzlich auch mit einem Spulstellenrechner 22 der jeweiligen Spulstelle 21.1 verbunden sein. Die Steuereinheit 14 weist eine Ausgabeeinheit und eine Eingabeeinheit für eine Bedienungsperson auf. Vorzugsweise sind Ausgabe- und Eingabeeinheit gemeinsam als Sensorbildschirm (Touchscreen) 15 ausgebildet. Die Steuereinheit 14 ist mit einem Steuerrechner 23 der Spulmaschine 2 verbunden. Statt in der Steuereinheit 14 können die Ausgabe- und/oder die Eingabeeinheit in der Spulmaschine 2, z. B. im Steuerrechner 23, eingebaut sein.

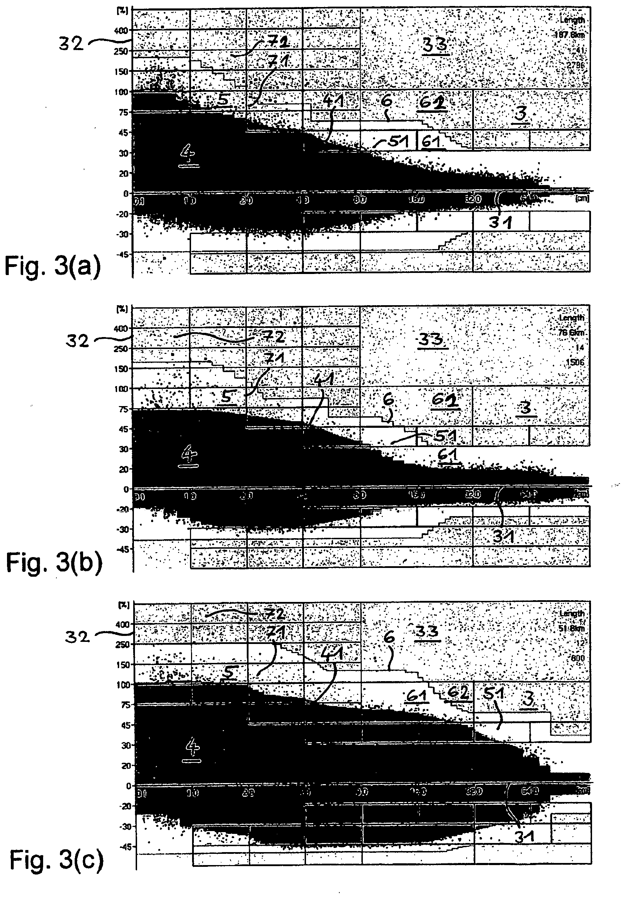

**Figur 2** zeigt ein mögliches Ereignisfeld 3 mit einem Garnkörper und einer Reinigungsgrenze 6, wie es auf der Ausgabeeinheit 15 der Steuereinheit 14 dargestellt werden kann. Das Ereignisfeld 3 beinhaltet Teile des ersten und vierten Quadranten eines zweidimensionalen kartesischen Koordinatensystems, das durch eine Abszisse 31 und eine Ordinate 32 aufgespannt wird. Die Elemente im vierten Quadranten tragen hier dieselben Bezugszeichen wie die analogen Elemente im ersten Quadranten, aber mit einem Strich versehen. Die Ordinate 32 gibt z. B. eine Abweichung ΔM des Garnparameters, z. B. die Abweichung der Garnmasse pro Längeneinheit, von einem Sollwert an. Der Sollwert wird vorzugsweise durch eine laufende Mittelwertbildung über eine Vielzahl von Messungen ermittelt. Die Abszisse 31 gibt an, über welche Länge L entlang der Garnlängsrichtung sich eine Abweichung ΔM erstreckt. Die Achsen 31, 32 für Länge und Massenzunahme bzw. Massenabnahme sind vorzugsweise logarithmisch, fast logarithmisch oder teilweise logarithmisch eingeteilt. Eine ermittelte Abweichung ΔM und ihre Länge L bilden zusammen die Koordinaten eines Garnereignisses, das einen Punkt im Ereignisfeld 3 definiert und dort auf geeignete Weise dargestellt werden kann (vgl. Figur 3). Im ersten Quadranten werden durch lokale Massenzunahme verursachte Dickstellen, im zweiten Quadranten durch lokale Massenabnahme verursachte Dünnstellen gegenüber der jeweiligen Länge L aufgetragen.

Es wird ein genügend langer Garnabschnitt, z. B. 100 km, ausgemessen. Die Werte des Garnparameters ΔM und die dazugehörigen Längen L werden vom Messkopf 11 an die Steuereinheit 14 übermittelt (vgl. Figur 1). In einer Recheneinheit der Steuereinheit 14 werden daraus Dichten von Ereignissen im Ereignisfeld 3 ermittelt, so wie es z. B. in der US-6,374,152 B1 beschrieben ist. Auf diese Weise kann jedem Punkt des Ereignisfeldes 3 eindeutig eine Ereignisdichte zugeordnet werden. Durch Interpolation, Extrapolation, Glättung und/oder andere numerische Verfahren können zu abrupte lokale Änderungen der so ermittelten Ereignisdichtefunktion, die möglicherweise durch Messfehler oder sonstige Artefakte verursacht sind, vermieden werden.

Aus der Ereignisdichtefunktion wird ein Garnkörper berechnet und als Fläche 4 im Ereignisfeld 3 dargestellt. Es wird eine genügend hohe Grenzereignisdichte von z. B. 1000 Ereignissen pro 100 km Garnlänge gewählt. Die Verbindung aller Punkte im Ereignisfeld 3, denen die Grenzereignisdichte zugeordnet ist, ergibt eine Dichtekurve 41, welche den Garnkörper vom übrigen Ereignisfeld 3 abgrenzt. Gegen die beiden Koordinatenachsen 31, 32 hin wird der Garnkörper durch die Koordinatenachsen 31, 32 selbst begrenzt. Durch diese Abgrenzungen entsteht eine zusammenhängende Fläche 4, die für das ausgemessene Garn 9 charakteristisch ist. Der Garnkörper ist bezüglich der Abszisse 31 nicht notwendigerweise symmetrisch. Die den Garnkörper darstellende Fläche 4 kann sich grafisch vom übrigen Ereignisfeld 3 unterscheiden, indem sie z. B. eine andere Farbe, einen anderen Grauton und/oder ein anderes Muster aufweist als das übrige Ereignisfeld 3.

Im Ereignisfeld 3 ist auch eine Reinigungskurve 6 für die Ausreinigung von Fehlern aus dem Garn 9 eingezeichnet. Die Reinigungskurve 6 ist diejenige Linie, welche das Ereignisfeld 3 in Reinigungsbereiche 61, 62 aufteilt, von denen ein erster Reinigungsbereich 61 zulässige Ereignisse und ein zweiter Reinigungsbereich 62 unzulässige Ereignisse vorgibt. Sie ist die grafische Darstellung einer Reinigungsgrenze, d. h. eines Kriteriums für die Beurteilung der Garnqualität. Sie liegt deutlich über dem Garnkörper 4, weil es unsinnig wäre, Ereignisse aus dem Garnkörper 4 ausreinigen zu wollen.

**Figur 3** zeigt erweiterte Darstellungen von Ereignisfeldern 3, wie sie auf der Ausgabeeinheit 15 der Steuereinheit 14 (vgl. Figur 1) ausgegeben werden können. In den Beispielen von Figur 3 schliesst sich jeweils an die den Garnkörper darstellende Fläche 4 eine weitere Fläche 5 an. Die weitere Fläche 5 wird durch die den Garnkörper begrenzende Dichtekurve 41 und durch eine weitere Dichtekurve 51, die einer tieferen Ereignisdichte entspricht, begrenzt. Die höhere Ereignisdichte kann z. B. 1000 Ereignisse pro 100 km Garnlänge und die tiefere Ereignisdichte z. B. 100 Ereignisse pro 100 km Garnlänge betragen. Diese beiden Ereignisdichten sind somit Teil der geometrischen Folge 1, 10, 100, 1000, ... Ereignisse pro 100 km Garnlänge. Die den Garnkörper darstellende Fläche 4 und die weitere Fläche 5 unterscheiden sich grafisch voneinander und von ihrer jeweiligen Umgebung. So können z. B. die den Garnkörper darstellende Fläche 4 dunkelgrün und die weitere Fläche 5 hellgrün eingefärbt sein. Statt nur einer weiteren Fläche 5 könnten auch mehrere weitere Flächen dargestellt werden. Solche weiteren Flächen 5 können der Bedienungsperson helfen, den Charakter des Garns 9 noch besser zu erfassen und die Reinigungsgrenze 6 optimal vorzugeben, falls die Reinigungsgrenze 6 nicht automatisch festgelegt wird.

In den Ereignisfeldern 3 von Figur 3 ist jeweils auch eine Reinigungsgrenze 6 eingezeichnet. Sie trennt einen ersten Reinigungsbereich 61, der zulässige Ereignisse vorgibt, und einen zweiten Reinigungsbereich 62, der unzulässige Ereignisse vorgibt, voneinander. Die beiden Reinigungsbereiche 61, 62 sind vorzugsweise unterschiedlich eingefärbt, z. B. der erste Reinigungsbereich weiss und der zweite Reinigungsbereich grau. Die Reinigungsgrenze 6 ist grundsätzlich von den Dichtekurven 41, 51 unabhängig, obwohl sie sich in ihrem groben Verlauf an sie halten wird. In Figur 3 fällt die Reinigungsgrenze 6 teilweise mit der weiteren Dichtekurve 51 zusammen, teilweise nicht.

Ferner sind in den Ereignisfeldern 3 von Figur 3 Ereignisse 71, 72 in Form von Quadraten eingezeichnet. Dabei können sich die Symbole für die zulässigen Ereignisse 71 im ersten Reinigungsbereich 61 diesseits der Reinigungsgrenze 6 von denjenigen für die unzulässigen Ereignisse 72 im zweiten Reinigungsbereich 62 jenseits der Reinigungsgrenze 6 unterscheiden, z. B. durch ihre Grösse und/oder ihre Farbe. Die Ereignisse bilden zusammen eine so genannte Punktewolke (englisch scatter plot).

Nicht alle gemessenen Ereignisse werden im Ereignisfeld 3 eingetragen. Der grösste Teil des Garnkörpers ist frei von Ereignissen. Grund dafür ist, dass Ereignisse im Garnkörper für eine Bedienungsperson schlicht uninteressant sind. Sie "gehören zum Garn". Das Nichtdarstellen derjenigen Ereignisse, die in der Nähe der Abszisse 31 liegen, hat zumindest die folgenden beiden Vorteile:
- Diese Ereignisse brauchen nicht vom Messkopf 11 zur Steuereinheit 14 übertragen zu werden. Somit wird die zu übertragende und zu verarbeitende Datenmenge wesentlich reduziert und die Datenleitungen sowie die Steuereinheit 14 entlastet. Die dadurch frei gewordenen Kapazitäten können für Wichtigeres genutzt werden.
- Die grafische Darstellung enthält weniger Ereignispunkte und wird dadurch übersichtlicher. Die Bedienungsperson wird nicht von unnötigen Ereignispunkten abgelenkt und kann sich auf das Wesentliche konzentrieren.

Die Ereignisfelder 3 sind also jeweils in zwei Klassierbereiche aufgeteilt: in einen ersten Klassierbereich, in dem keine Ereignisse dargestellt werden, und in einen zweiten Klassierbereich, in dem Ereignisse dargestellt werden. Die beiden Klassierbereiche sind durch eine Klassiergrenze voneinander abgegrenzt, die jedoch im Beispiel von Figur 3 nicht eingezeichnet ist. Sie folgt vorzugsweise auch einer Dichtekurve, die einer höheren, gleichen oder niedrigeren Ereignisdichte entsprechen kann als die den Garnkörper 4 begrenzende Dichtekurve 41.

In einem Beschreibungsfeld 33 findet man in Figur 3(a) die folgenden beispielhaften Angaben:
- Die Garnlänge, aufgrund derer die Dichtekurven 41, 42 für den Garnkörper 4 und die weitere Fläche 5 ermittelt wurden, betrug 187.8 km.
- Die Anzahl Ereignisse 72, die in den beiden zweiten Reinigungsbereichen 62 liegen und somit als Fehler aus dem Garnabschnitt von 187.8 km Länge entfernt würden, beträgt 41. Somit ergäben sich 22 Schnitte pro 100 km Garnlänge, welche Schnittzahl bei Bedarf ebenfalls ausgegeben werden könnte.
- Die Gesamtzahl im Ereignisfeld 3 eingetragener Ereignisse 71, 72 beträgt 2796.

In den Beschreibungsfeldern 33 der Figuren 3(b) und (c) sind analoge Daten angegeben.

Die drei Beispiele der Figuren 3(a)-(c) beziehen sich auf verschiedene Garntypen gemäss der folgenden Tabelle:

| **Figur** | **Rohmaterial** | **Garnnummer (Nec)** |
|---|---|---|
| 3(a) | Baumwolle | 30 (20 tex) |
| 3(b) | Baumwolle | 10 (60 tex) |
| 3(c) | Schurwolle | 30 (20 tex) |

Wie ein Vergleich der drei Beispiele von Figur 3 zeigt, unterscheiden sich die Garnkörper für verschiedene Garntypen in qualitativer und quantitativer Hinsicht:
- In Figur 3(a) ist der Garnkörper im ersten Quadranten für ganz kleine Fehlerlängen L < 1 cm auf einem relativ hohen Wert von ca. 100 % und nimmt gegen grössere Fehlerlängen L hin monoton ab. Auffällig ist die grosse Anzahl Nissen (sehr kurze Fehlerlänge L, sehr grosse Massenabweichung ΔM). Im vierten erreicht der Garnkörper einen Minimalwert von ca. -30 % für Fehlerlängen L zwischen 2 cm und 4 cm.
- In Figur 3(b) ist der Garnkörper im ersten Quadranten für ganz kleine Fehlerlängen L < 1 cm auf einem niedrigeren Wert von ca. 75 %. Gegen grössere Fehlerlängen L bleibt er annähernd konstant und nimmt erst ab ca. L = 4 cm markant ab, um dann ab ca. L = 16 cm konstant zu bleiben. Im vierten hat der Garnkörper einen ähnlichen Verlauf wie derjenige von Figur 3(a).
- In Figur 3(c) beginnt der Garnkörper im ersten Quadranten auf einem relativ hohen Wert von ca. 100 % und bleibt zunächst auf diesem hohen Wert konstant. Eine kleinere, stufenförmige Abnahme erfolgt bei ca. L = 2 cm, eine weitere, starke Abnahme erst ab ca. L = 16 cm. Im vierten Quadranten liegt für Fehlerlängen L zwischen ca. 2 cm und 16 cm ein langes Minimalplateau auf einem relativ tiefen Wert von ca. -45 % vor. Insgesamt weist der Garnkörper von Figur 3(c) eine grössere Fläche auf als diejenigen der Figuren 3(a) und (b).

Anhand von Charakteristika eines durch Messung bestimmten Garnkörpers, wie sie oben beschrieben sind, kann erfindungsgemäss automatisch auf den Garntyp geschlossen werden. Das heisst, dass die Steuereinheit 14 aufgrund von Messwerten und vorgängig eingegebenen oder gelernten Garncharakteristika automatisch bestimmen kann, um welchen Garntyp es sich beim ausgemessenen Garn handelt.

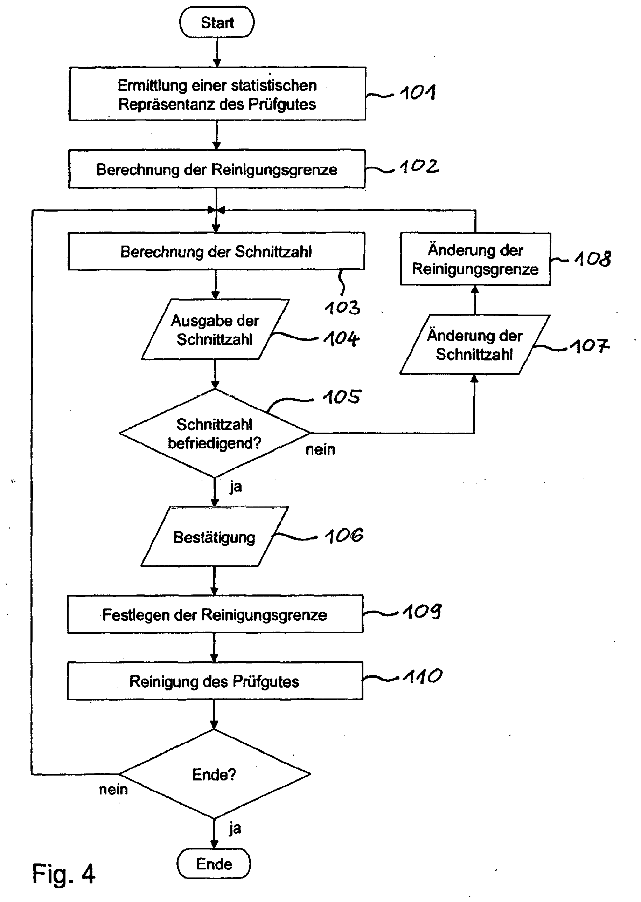

**Figur 4** zeigt ein Flussdiagramm einer bevorzugten Ausführungsform des erfindungsgemässen Verfahrens. Das Verfahren wird auf möglichst einfache Weise gestartet. In einer bevorzugten Ausführungsform ist dazu auf einer Bedienungsoberfläche ein Startknopf vorhanden, der z. B. mit "Smart limits", "Auto setup" oder ähnlich angeschrieben sein kann. Der Startknopf kann hardwaremässig oder softwaremässig realisiert sein. Im letzteren Fall kann er symbolisch auf einem Bildschirm dargestellt und kann mittels einer Eingabeeinheit wie einer Tastatur oder einer Computermaus, oder durch Berührung, falls es sich um einen Sensorbildschirm 15 (siehe Figur 1) handelt, betätigt werden.

In einem Einmessvorgang 101 wird eine statistische Repräsentanz des Prüfgutes ermittelt. Bei der statistischen Repräsentanz handelt es sich bspw. um eine Punktewolke von Ereignispunkten 71, 72, wie sie in Figur 3 dargestellt ist. Die statistische Repräsentanz wird vorzugsweise erfasst, indem ein oder mehrere Messköpfe 11 einen genügend langen Abschnitt des Prüfgutes 9, z. B. 100 km Garn, vermessen und die Messdaten in der Steuereinheit 14 verarbeitet werden.

Aufgrund der erfassten statistischen Repräsentanz wird automatisch eine Reinigungsgrenze für das Prüfgut berechnet 102. Dabei handelt es sich vorzugsweise um eine von der Steuereinheit 14 berechnete und vorgeschlagene Reinigungskurve 6. Sie kann z. B. als eine Dichtekurve, d. h. als Menge aller Orte im Ereignisfeld 3 mit konstanter Ereignisdichte, berechnet werden. Eine solche Berechnung der Reinigungskurve 6 ist also analog zur Berechnung der Grenzereignisdichte 41, welche den Garnkörper 4 vom übrigen Ereignisfeld 3 abgrenzt (siehe Figuren 2 und 3), doch hat die Reinigungskurve 6 eine deutlich tiefere Ereignisdichte als die Grenzereignisdichte 41 für den Garnkörper 4, liegt also über dem Garnkörper 4. Die Ereignisdichte für die Reinigungskurve 6 hängt davon ab, welche Schnittzahl gewünscht wird. Im einfachsten Fall kann die Anfangsschnittzahl ein fest vorgegebener Wert wie z. B. 50 Schnitte/100 km sein. Falls der Bedienungsperson eine gewisse Auswahlmöglichkeit zur Verfügung gestellt werden soll, können ihr einige einfache Auswahlmöglichkeiten für die Wahl der Anfangsschnittzahl angeboten werden, z. B.:
- "Niedrig" für niedrige Schnittzahl (z. B. 10 Schnitte/100 km) oder niedrige Qualität,
- "Mittel" für mittlere Schnittzahl (z. B. 50 Schnitte/100 km) oder mittlere Qualität und
- "Hoch" für hohe Schnittzahl (z. B. 100 Schnitte/100 km) oder hohe Qualität.

Zur Auswahl können hardwaremässig oder softwaremässig realisierte Auswahlknöpfe vorgesehen sein. Für eine völlige Wahlfreiheit kann der Bedienungsperson die Möglichkeit der freien Eingabe einer gewünschten Schnittzahl gewährt werden. Bei der Entscheidung für eine der obigen Optionen sollte eine Abwägung zwischen der Freiheit der Bedienungsperson einerseits und der Einfachheit der Bedienung andererseits gemacht werden.

Nebst der statistischen Repräsentanz können aber auch weitere Einflüsse in die Berechnung der Reinigungsgrenze einfliessen. Solche weiteren Einflüsse können z. B. die folgenden sein:
- Vergangenheit des Prüfgutes 9. Für den Verlauf der Reinigungsgrenze 6 kann es eine Rolle spielen, aus welchen Rohmaterialien das Prüfgut 9 hergestellt wurde und welche Verarbeitungsprozesse es durchlaufen hat. Wie Figur 3 und ihre obige Diskussion zeigt, hat die Vergangenheit einen Einfluss auf den Garnkörper 4. Dementsprechend kann sie auch bei der Festlegung der Reinigungsgrenze 6 berücksichtigt werden.

- Gegenwart des Prüfgutes 9. Der gegenwärtige Zustand des Prüfgutes 9 kann durch Parameter wie Garnnummer, Ungleichmässigkeit, Fremdstoffe, Feuchtegehalt etc. charakterisiert werden. Einige dieser Parameter werden möglicherweise während des Einmessvorgangs 101 erfasst, andere nicht. Die letzteren Parameter oder zumindest einige davon können durch die Bedienungsperson in die Steuereinheit 14 eingegeben werden.
- Zukunft des Prüfgutes 9. Die zukünftige Verwendung des Prüfgutes 9 kann die Reinigungsgrenze 6 beeinflussen. So sind etwa bei einem Schussgarn für ein Gewebe kurze Massenschwankungen eher störend als lange, während bei einem Garn für ein Gestrick das Gegenteil der Fall ist.
- Produktions- und Messanlage. So kann z. B. der Typ des Messkopfes 11, mit dem das Prüfgut 9 abgetastet wird, einen Einfluss auf die Verteilung der Ereignisse 71, 72 im Ereignisfeld 3 haben. Ein solcher Einfluss kann bei der automatischen Festlegung der Reinigungsgrenze 6 kompensiert werden.
- Umgebungsbedingungen. Luftfeuchtigkeit, Lufttemperatur und andere Umgebungsbedingungen können ebenfalls die Verteilung der Ereignisse 71, 72 im Ereignisfeld 3 beeinflussen. Eine Kompensation ist möglich.

Wenn also z. B. bekannt ist, dass das betreffende Garn 9 als Strickgarn verwendet werden wird, so kann dies auf geeignete Weise in die Steuereinheit 14 eingegeben werden. Bei der automatischen Festlegung der Reinigungsgrenze 6 wird daraufhin die Reinigungsgrenze 6 für lange Fehler tiefer und für kurze Fehler höher gelegt als die entsprechende Dichtelinie.

Aufgrund der statistischen Repräsentanz und der Reinigungsgrenze 6 wird automatisch eine auf die Prüfgutlänge bezogene Anzahl Schnitte berechnet 103. Diese längenbezogene Schnittzahl ergibt sich im einfachsten Fall aus der Summe aller über der Reinigungskurve 6 liegenden Ereignisse 72. Sie wird meistens auf eine Prüfgutnormlänge von 100 km umgerechnet. Komplizierter wird es, wenn mehrere voneinander zunächst unabhängige Messwerte am Prüfgut 9 erfasst werden und für jeden Messwert eine separate Teilreinigung erfolgt. Ein typisches Beispiel für den letztgenannten Fall ist die Teilreinigung nach Massen- oder Durchmesserschwankungen (NSLT-Reinigung) einerseits und die Teilreinigung nach Fremdstoffen andererseits. In diesem Fall liefert jede der verschiedenen Teilreinigungen eine Teilschnittzahl, und die Gesamtschnittzahl ergibt sich aus der Summe aller Teilschnittzahlen. Die Verhältnisse der Teilschnittzahlen können untereinander geändert werden, ohne die Gesamtschnittzahl zu ändern. Dadurch ergeben sich zusätzliche Freiheitsgrade bezüglich der Festlegung der einzelnen Teilreinigungsgrenzen. Die Verhältnisse der Teilschnittzahlen können entweder durch eine Bedienungsperson eingegeben, fest vorgegeben oder automatisch berechnet werden. Die automatische Berechnung kann wiederum auf den oben diskutierten weiteren Einflüssen basieren. So kann es z. B. zukünftige Verwendungen des Prüfgutes geben, in denen Fremdstoffe wenig stören, Massenschwankungen hingegen schon.

Die Bedienungsperson muss die Möglichkeit haben, sich zur Schnittzahl, die zur festgelegten Reinigungsgrenze 6 gehört, zu äussern 105. Zu diesem Zweck wird die Schnittzahl nach ihrer Berechnung 103 auf einer Ausgabeeinheit 15 angezeigt. Die Bedienungsperson wird aufgefordert, die angezeigte Schnittzahl zu bestätigen oder zu ändern. Zu diesem Zweck kann auf der Steuereinheit 14 ein Bedienungsfeld 200 zur Verfügung gestellt werden. Ein Beispiel für ein solches Bedienungsfeld 200 ist in **Figur 5** dargestellt. Darin wird in einem ersten Zahlenfeld 201 die aktuelle Schnittzahl, bspw. 52.5 Schnitte/100 km, angezeigt und bei Bedarf auch eingegeben. Die Schnittzahl kann mittels inkrementaler Tasten 205, 206 um ein Inkrement, z. B. 10 Schnitte/100 km, geändert werden 107 (vgl. Figur 4). Zur Anzeige und bei Bedarf zur Eingabe des Inkrementes ist ein zweites Zahlenfeld 202 vorgesehen. Das Inkrement kann automatisch berechnet oder vorgeschlagen werden, vorzugsweise als ein bestimmter Bruchteil, z. B. 20 %, der Schnittzahl. Durch Drücken der ersten inkrementalen Taste 205 wird die Reinigungskurve 6, 6' weiter vom Garnkörper 4, 4' entfernt (vgl. Figur 2), wodurch die Schnittzahl erniedrigt wird; umgekehrt bewirkt ein Drücken der zweiten inkrementalen Taste 206 eine Annäherung der Reinigungskurve 6, 6' an den Garnkörper 4, 4' und somit eine Erhöhung der Schnittzahl. Ein drittes Zahlenfeld 203 kann die Länge des bereits gespulten Garns anzeigen, und in einem vierten Feld 204 kann eine Warnung ausgegeben werden, wenn diese Länge einen empfohlenen Grenzwert, z. B. 100 km, unterschreitet. Ferner ist ein Bestätigungsknopf 207 zur Bestätigung 106 (vgl. Figur 4) der aktuellen Reinigungsgrenze und Schnittzahl vorhanden. Mit einem Verwerfknopf 208 können alle bisherigen Änderungen verworfen werden. Aus dem Gesagten geht hervor, dass im Ausführungsbeispiel von Figur 5 die Bestätigung 106 bzw. der Änderungsbefehl 107 durch nur ein einziges Drücken oder Anwählen eines hardwaremässig oder softwaremässig realisierten Betätigungsknopfes 205, 206 oder 207 erfolgt. Dies macht die Bedienung besonders einfach. Selbstverständlich brauchen nicht alle in Figur 5 dargestellten Felder und Knöpfe in einem Bedienungsfeld 200 beieinander zu liegen. Stattdessen können sie auf andere Weise auf der Eingabe- und/oder Ausgabeeinheit 15 (Figur 1) verteilt sein. Für das Funktionieren der Erfindung sind auch nicht alle beschriebenen Elemente 201-208 des Bedienungsfeldes nötig.

Aufgrund des eingegebenen Änderungsbefehls für die Schnittzahl wird die Reinigungsgrenze 6 automatisch geändert 108. Eine zur geänderten Reinigungsgrenze gehörige neue Schnittzahl wird berechnet 103. Dabei wird von der bereits früher ermitteleten statistischen Repräsentanz ausgegangen. Zusätzlich können aber weitere Einflüsse berücksichtigt werden, wie dies schon anlässlich der anfänglichen Festlegung 102 der Reinigungsgrenze 6 beschrieben wurde. Der Bedienungsperson wird wiederum Gelegenheit gegeben 105, sich zur neuen Schnittzahl zu äussern und sie gegebenenfalls zu ändern 107. Die beschriebene Schleife zur Optimierung der Reinigungsgrenze 6 bzw. der Schnittzahl kann so oft durchlaufen werden, bis die Bedienungsperson zufrieden ist und bestätigt 106. Erst dann wird die Reinigungsgrenze 6 festgelegt.

Die auf diese Weise festgelegte und möglicherweise optimierte Reinigungsgrenze 6 wird von der Steuereinheit 14 an die Messköpfe 11 übermittelt und an diesen eingestellt, wodurch sie festgelegt wird 109. Mit dieser Einstellung wird Prüfgut 9 gereinigt 110. Dabei kann es vorteilhaft sein, die Schnittzahl neu zu berechnen und periodisch, bei besonderen Ereignissen wie einer markanten Änderung der Schnittzahl und/oder auf Eingabe einer Bedienungsperson hin eine erneute Überprüfung 105 und bei Bedarf Änderung 107 der Schnittzahl erfolgen.

### BEZUGSZEICHENLISTE

- 1: Reinigungsanlage
- 11: Messkopf
- 12: Schnittstellenwandler
- 13: Verbindungsleitung
- 14: Steuereinheit
- 15: Sensorbildschirm als Eingabe- und Ausgabeeinheit

- 2: Spulmaschine
- 21.1, 21.2, ...: Spulstellen
- 22: Spulstellenrechner
- 23: Steuerrechner

- 3: Ereignisfeld
- 31: Abszisse
- 32: Ordinate

- 4: Prüfgutkörper, die den Garnkörper darstellende Fläche
- 41: das Ereignisfeld abgrenzende Dichtekurve

- 5: weitere Fläche
- 51: die weitere Fläche abgrenzende Dichtekurve

- 6: Reinigungsgrenze
- 61: erster Reinigungsbereich
- 62: zweiter Reinigungsbereich

- 71: zulässiges Ereignis
- 72: unzulässiges Ereignis

- 9: Prüfgut, z. B. Garn

- 101: Ermittlung einer statistischen Repräsentanz
- 102: Berechnung der Reinigungsgrenze
- 103: Berechnung der Schnittzahl
- 104: Ausgabe der Schnittzahl
- 105: Entscheidung: Schnittzahl befriedigend?
- 106: Bestätigung der Schnittzahl
- 107: Änderung der Schnittzahl
- 108: Änderung der Reinigungsgrenze
- 109: Festlegen der Reinigungsgrenze
- 110: Reinigung des Prüfgutes

- 200: Bedienungsfeld
- 201-204: Zahlenfelder
- 205, 206: inkrementale Tasten
- 207: Bestätigungstaste
- 208: Verwerftaste

## Patentansprüche

1. Verfahren zum Festlegen einer Reinigungsgrenze (6) auf einer elektronischen Reinigungsanlage (1) für ein längliches textiles Prüfgut (9) wie Garn, wobei Messwerte mindestens einer Eigenschaft des entlang seiner Längsrichtung bewegten Prüfgutes (9) auf einer bestimmten Prüfgutlänge erfasst werden,
aus den Messwerten Ereignisse (71, 72) im Prüfgut (9) identifiziert werden,
eine statistische Repräsentanz des Prüfgutes (9) aus den Ereignissen (71, 72) ermittelt wird (101),
aufgrund der statistischen Repräsentanz dem Prüfgut (9) automatisch ein bestimmter Prüfguttyp zugeordnet wird,
eine Reinigungsgrenze (6) als Kriterium für die Zulässigkeit bzw. Unzulässigkeit der Ereignisse (71, 72) aufgrund der statistischen Repräsentanz berechnet wird (102), wobei der zugeordnete Prüfguttyp die Berechnung (102) der Reinigungsgrenze (6) beeinflusst,
eine längenbezogene Anzahl unzulässiger Ereignisse (72) aus der ermittelten statistischen Repräsentanz und der Reinigungsgrenze (6) berechnet wird (103),
die längenbezogene Anzahl unzulässiger Ereignisse (72) auf einer Ausgabeeinheit (15) ausgegeben wird,
eine Bedienungsperson aufgefordert wird (105), eine Stellungnahme zur ausgegebenen längenbezogenen Anzahl unzulässiger Ereignisse (72) mittels einer Eingabeeinheit (15) einzugeben und
die Reinigungsgrenze (6) gemäss der eingegebenen Stellungnahme automatisch festgelegt wird (109).

2. Verfahren zum Festlegen einer Reinigungsgrenze (6) auf einer elektronischen Reinigungsanlage (1) für ein längliches textiles Prüfgut (9) wie Garn, wobei Messwerte mindestens einer Eigenschaft des entlang seiner Längsrichtung bewegten Prüfgutes (9) auf einer bestimmten Prüfgutlänge erfasst werden,
aus den Messwerten Ereignisse (71, 72) im Prüfgut (9) identifiziert werden,
eine statistische Repräsentanz des Prüfgutes (9) aus den Ereignissen (71, 72) ermittelt wird (101),
eine Reinigungsgrenze (6) als Kriterium für die Zulässigkeit bzw. Unzulässigkeit der Ereignisse (71, 72) aufgrund der statistischen Repräsentanz berechnet wird (102), wobei eine für die Zukunft vorgesehene Verwendung des Prüfgutes (9) die Berechnung (102) der Reinigungsgrenze (6) beeinflusst,
eine längenbezogene Anzahl unzulässiger Ereignisse (72) aus der ermittelten statistischen Repräsentanz und der Reinigungsgrenze (6) berechnet wird (103), die längenbezogene Anzahl unzulässiger Ereignisse (72) auf einer Ausgabeeinheit (15) ausgegeben wird,
eine Bedienungsperson aufgefordert wird (105), eine Stellungnahme zur ausgegebenen längenbezogenen Anzahl unzulässiger Ereignisse (72) mittels einer Eingabeeinheit (15) einzugeben und
die Reinigungsgrenze (6) gemäss der eingegebenen Stellungnahme automatisch festgelegt wird (109).

3. Verfahren zum Festlegen einer Reinigungsgrenze (6) auf einer elektronischen Reinigungsanlage (1) für ein längliches textiles Prüfgut (9) wie Garn, wobei Messwerte mehrerer Eigenschaften des entlang seiner Längsrichtung bewegten Prüfgutes (9) auf einer bestimmten Prüfgutlänge erfasst werden,
aus den Messwerten Ereignisse (71, 72) im Prüfgut (9) identifiziert werden, für jede der Eigenschaften aus den Ereignissen (71, 72) eine statistische Repräsentanz des Prüfgutes (9) ermittelt wird,
für jede der Eigenschaften eine Reinigungsgrenze (6) als Kriterium für die Zulässigkeit bzw. Unzulässigkeit der Ereignisse (71, 72) berechnet wird (102), für jede der Eigenschaften eine sich aus der ermittelten statistischen Repräsentanz und der Reinigungsgrenze (6) ergebende längenbezogene Teilanzahl unzulässiger Ereignisse (72) berechnet wird,
eine längenbezogene Gesamtanzahl unzulässiger Ereignisse (72) als Summe der Teilanzahlen berechnet wird,
Verhältnisse der Teilanzahlen, vorzugsweise automatisch, derart festgelegt werden, dass die Gesamtanzahl unzulässiger Ereignisse (72) unverändert bleibt,
für jede der Eigenschaften die Reinigungsgrenze (6) derart berechnet wird, dass sich aus der ermittelten statistischen Repräsentanz und der berechneten Reinigungsgrenze (6) die festgelegte längenbezogene Teilanzahl unzulässiger Ereignisse (72) ergibt,
die längenbezogene Gesamtanzahl unzulässiger Ereignisse (72) auf einer Ausgabeeinheit (15) ausgegeben wird,
eine Bedienungsperson aufgefordert wird (105), eine Stellungnahme zur ausgegebenen längenbezogenen Gesamtanzahl unzulässiger Ereignisse (72) mittels einer Eingabeeinheit (15) einzugeben und
die Reinigungsgrenze (6) gemäss der eingegebenen Stellungnahme automatisch festgelegt wird (109).

4. Verfahren nach einem der vorangehenden Ansprüche, wobei die Stellungnahme entweder eine Bestätigung (106) ist, worauf die Reinigungsgrenze (6) unverändert belassen wird,
oder ein Änderungsbefehl (107) ist, worauf eine neue Reinigungsgrenze (6) gemäss dem Änderungsbefehl und aufgrund der statistischen Repräsentanz berechnet wird (108) und eine neue längenbezogene Anzahl unzulässiger Ereignisse (72) aus der ermittelten statistischen Repräsentanz sowie der neuen Reinigungsgrenze (6) berechnet und auf der Ausgabeeinheit (15) ausgegeben wird.

5. Verfahren nach Anspruch 4, wobei der Änderungsbefehl entweder ein Befehl zur Erhöhung der längenbezogenen Anzahl unzulässiger Ereignisse (72) oder ein Befehl zur Erniedrigung der längenbezogenen Anzahl unzulässiger Ereignisse (72) ist.

6. Verfahren nach Anspruch 5, wobei die Erhöhung bzw. Erniedrigung der längenbezogenen Anzahl unzulässiger Ereignisse (72) um einen automatisch berechneten Inkrementwert erfolgt, der im Wesentlichen proportional zur berechneten längenbezogenen Anzahl unzulässiger Ereignisse (72) ist.

7. Verfahren nach einem der Ansprüche 4-6, wobei die Bestätigung (106) bzw. der Änderungsbefehl (107) durch eine einzige Handlung, bspw. einen einzigen Knopfdruck, erfolgt.

8. Verfahren nach einem der Ansprüche 4-7, wobei nach einem Änderungsbefehl die Bedienungsperson wiederum aufgefordert wird (105), eine Stellungnahme zur ausgegebenen neuen längenbezogenen Anzahl unzulässiger Ereignisse (72) mittels der Eingabeeinheit (15) einzugeben und die Reinigungsgrenze (6) gemäss der eingegebenen Stellungnahme automatisch festgelegt wird (108).

9. Verfahren nach einem der vorangehenden Ansprüche, wobei die Reinigungsgrenze (6) aufgrund der statistischen Repräsentanz derart berechnet wird (102), dass eine sich aus der ermittelten statistischen Repräsentanz und der Reinigungsgrenze (6) ergebende längenbezogene Anzahl unzulässiger Ereignisse (72) pro 100 km Prüfgutlänge zwischen 1 und 200, vorzugsweise zwischen 10 und 100, liegt.

10. Verfahren nach einem der vorangehenden Ansprüche, wobei zusätzlich zur besagten Reinigungsgrenze (6) eine Spleissreinigungsgrenze als Kriterium für zulässige bzw. unzulässige Spleisse im Prüfgut (9) festgelegt wird und die besagte Reinigungsgrenze (6) so festgelegt wird, dass sie immer oberhalb der Spleissreinigungsgrenze liegt.

11. Verfahren nach einem der vorangehenden Ansprüche, wobei die festgelegte Reinigungsgrenze (6) automatisch auf mindestens ein vorgegebenes Kriterium hin überprüft wird und bei Nichterfüllung des Kriteriums eine Warnung ausgegeben wird.

12. Verfahren nach einem der vorangehenden Ansprüche, wobei
die Ereignisse (71, 72) durch die Messwerte (M) einerseits und durch eine Erstreckung (L) von Messwerten in der Längsrichtung andererseits definiert werden, ein Ereignisfeld (3) bereitgestellt wird, das einen Quadranten oder einen Teil eines Quadranten eines zweidimensionalen kartesischen Koordinatensystems beinhaltet, dessen Abszisse (31) die Erstreckung (L) der Messwerte in der Längsrichtung und dessen Ordinate (32) eine Abweichung (ΔM) der Messwerte von einem Sollwert angibt,
aus den Messwerten und ihrer Erstreckung (L) in der Längsrichtung Dichten von Ereignissen (71, 72) in dem Ereignisfeld (3) ermittelt werden, und
die Reinigungsgrenze (6) im Wesentlichen einer konstanten Ereignisdichte folgt.

13. Verfahren nach einem der vorangehenden Ansprüche, wobei die festgelegte Reinigungsgrenze (6) an mindestens einem Messkopf (11) der elektronischen Reinigungsanlage (1) eingestellt wird.

14. Verfahren nach einem der vorangehenden Ansprüche, wobei die Festlegung (102) der Reinigungsgrenze (6) automatisch erfolgt und durch eine einzige Handlung, bspw. einen einzigen Knopfdruck, einen einzigen Mausklick oder einen einzige Berührung eines Sensorbildschirms, ausgelöst wird.

15. Elektronische Reinigungsanlage (1) für ein längliches textiles Prüfgut (9) wie Garn, beinhaltend
mindestens einen Messkopf (11) zur Erfassung von Messwerten mindestens einer Eigenschaft des entlang seiner Längsrichtung bewegten Prüfgutes (9) auf einer bestimmten Prüfgutlänge und
eine mit dem Messkopf (11) verbundene Steuereinheit (14),
**dadurch gekennzeichnet, dass**
die Steuereinheit (14) zur Steuerung des Verfahrens nach einem der vorangehenden Ansprüche eingerichtet ist.

## Claims

1. A method for setting a clearing limit (6) on an electronic clearing system (1) for an elongated textile test material (9) such as yarn, wherein
measured values of at least one property of the test material (9) moved along its longitudinal direction are detected on a specific test material length,
events (71, 72) in the test material (9) are identified from the measured values,
a statistical representation of the test material (9) is established (101) from the events (71, 72),
the test material (9) is automatically assigned a specific test material type on the basis of the statistical representation,
a clearing limit (6) is calculated (102) as a criterion for the permissibility or impermissibility of the events (71, 72) on the basis of the statistical representation, wherein the assigned type of test material influences the calculation (102) of the clearing limit (6),
a length-related number of impermissible events (72) is calculated (103) from the established statistical representation and the clearing limit (6),
the length-related number of impermissible events (72) is output on an output unit (15),
an operator is requested (105) to enter a comment on the output length-related number of impermissible events (72) by means of an input unit (15), and
the clearing limit (6) is set automatically (109) according to the entered comment.

2. A method for setting a clearing limit (6) on an electronic clearing system (1) for an elongated textile test material (9) such as yarn, wherein
measured values of at least one property of the test material (9) moved along its longitudinal direction are detected on a specific test material length,
events (71, 72) in the test material (9) are identified from the measured values,
a statistical representation of the test material (9) is established (101) from the events (71, 72),
a clearing limit (6) is calculated (102) as a criterion for the permissibility or impermissibility of the events (71, 72) on the basis of the statistical representation,
wherein a future intended use of the test material (9) influences the calculation (102) of the clearing limit (6),
a length-related number of impermissible events (72) is calculated (103) from the established statistical representation and the clearing limit (6),
the length-related number of impermissible events (72) is output on an output unit (15),
an operator is requested (105) to enter a comment on the output length-related number of impermissible events (72) by means of an input unit (15), and
the clearing limit (6) is set automatically (109) according to the entered comment.

3. A method for setting a clearing limit (6) on an electronic clearing system (1) for an elongated textile test material (9) such as yarn, wherein
measured values of several properties of the test material (9) moved along its longitudinal direction are detected on a specific test material length,
events (71, 72) in the test material (9) are identified from the measured values,
a statistical representation of the test material (9) is established (101) for each of the properties from the events (71, 72),
a clearing limit (6) is calculated (102) for each of the properties as a criterion for the permissibility or impermissibility of the events (71, 72),
a length-related number of impermissible events (72) is calculated (103) for each of the properties from the established statistical representation and the clearing limit (6),
a length-related total number of impermissible events (72) is calculated as a sum total of the partial numbers,
ratios of the partial numbers are determined, preferably automatically, in such a way that the total number of impermissible events (72) remains unchanged,
the clearing limit (6) is calculated for each of the properties in such a way that the established length-related partial number of impermissible events (72) is obtained from the determined statistical representation and the calculated clearing limit (6), the length-related total number of impermissible events (72) is output on an output unit (15),
an operator is requested (105) to enter a comment on the output length-related total number of impermissible events (72) by means of an input unit (15), and
the clearing limit (6) is set automatically (109) according to the entered comment.

4. The method according to one of the preceding claims, wherein the comment is either a confirmation (106), whereupon the clearing limit (6) is left unchanged,
or a change command (107), whereupon a new clearing limit (6) is calculated (108) according to the change command and on the basis of the statistical representation, and a new length-related number of impermissible events (72) is calculated from the established statistical representation and the new clearing limit (6), and is output on the output unit (15).

5. The method according to claim 4, wherein the change command is either a command for increasing the length-related number of impermissible events (72) or a command for reducing the length-related number of impermissible events (72).

6. The method according to claim 5, wherein the increase or reduction of the length-related number of impermissible events (72) occurs by an automatically calculated incremental value which is substantially proportional to the calculated length-related number of impermissible events (72).

7. The method according to one of the claims 4 to 6, wherein the confirmation (106) or change command (107) occurs by a single action such as the single pressing of a button.

8. The method according to one of the claims 4 to 7, wherein after a change command the operator is requested again (105) to enter an comment concerning the output new length-related number of impermissible events (72) by means of the input unit (15), and the clearing limit (6) is automatically set (108) according to the entered comment.

9. The method according to one of the preceding claims, wherein the clearing limit (6) is calculated (102) on the basis of the statistical representation in such a way that a length-related number of impermissible events (72), which is derived from the established statistical representation and the clearing limit (6), lies between 1 and 200, preferably between 10 and 100, per 100 km of test material length.

10. The method according to one of the preceding claims, wherein a splice clearing limit is set in addition to the said clearing limit (6) as a criterion for permissible or impermissible splices in the test material (9), and the said clearing limit (6) is set in such a way that it always lies above the splice clearing limit.

11. The method according to one of the preceding claims, wherein the set clearing limit (6) is checked automatically with respect to at least one predetermined criterion and a warning is output in case of non-fulfillment of the criterion.

12. The method according to one of the preceding claims, wherein the events (71, 72) are defined by the measured values (M) on the one hand and by an extension (L) of measured values in the longitudinal direction on the other hand.

13. The method according to one of the preceding claims, wherein the set clearing limit (6) is established in at least one measuring head (11) of the electronic clearing system (1).

14. The method according to one of the preceding claims, wherein the setting (102) of the clearing limit (6) occurs automatically and is triggered by a single action such as for instance the single pressing of a button, a single mouse click or a single touch of a touchscreen.

15. An electronic clearing system (1) for an elongated textile test material (9) such as yarn, comprising
at least one measuring head (11) for detecting measured values of at least one property of the test material (9) over a specific length of the test material which is moved along its longitudinal direction, and
a control unit (14) connected with the measuring head (11),
**characterized in that**
the control unit (14) is set up for controlling the method according to one of the preceding claims.

## Revendications

1. Procédé pour fixer une limite de nettoyage (6) sur une installation de nettoyage électronique (1) pour un échantillon textile allongé (9) tel qu'un fil, dans lequel
des valeurs de mesure d'au moins une propriété de l'échantillon (9) déplacé dans le sens de sa longueur sont captées sur une longueur d'échantillon donnée,
des événements (71, 72) dans l'échantillon (9) sont identifiés à partir des valeurs de mesure,
une représentativité statistique de l'échantillon (9) est déterminée (101) à partir des événements (71, 72),
un certain type d'échantillon est automatiquement attribué à l'échantillon (9) sur la base de la représentativité statistique,
une limite de nettoyage (6) constituant un critère d'admissibilité ou de non admissibilité des événements (71, 72) est calculée sur la base de la représentativité statistique (102), le type d'échantillon affecté influençant le calcul (102) de la limite de nettoyage (6),
un nombre d'événements non admissibles rapporté à la longueur (72) est calculé (103) à partir de la représentativité statistique déterminée et de la limite de nettoyage (6),
le nombre d'événements non admissibles rapporté à la longueur (72) est émis sur une unité de sortie (15),
un opérateur est invité (105) à saisir un avis sur le nombre d'événements non admissibles rapporté à la longueur (72) émis en sortie au moyen d'une unité de saisie (15) et
la limite de nettoyage (6) est fixée automatiquement (109) suivant l'avis saisi.

2. Procédé pour fixer une limite de nettoyage (6) sur une installation de nettoyage électronique (1) pour un échantillon textile allongé (9) tel qu'un fil, dans lequel des valeurs de mesure d'au moins une propriété de l'échantillon (9) déplacé dans le sens de sa longueur sont captées sur une longueur d'échantillon donnée,
des événements (71, 72) dans l'échantillon (9) sont identifiés à partir des valeurs de mesure,
une représentativité statistique de l'échantillon (9) est déterminée (101) à partir des événements (71, 72),
une limite de nettoyage (6) constituant un critère d'admissibilité ou de non admissibilité des événements (71, 72) est calculée sur la base de la représentativité statistique (102), une utilisation future prévue de l'échantillon (9) influençant le calcul (102) de la limite de nettoyage (6),
un nombre d'événements non admissibles rapporté à la longueur (72) est calculé (103) à partir de la représentativité statistique déterminée et de la limite de nettoyage (6),
le nombre d'événements non admissibles rapporté à la longueur (72) est émis sur une unité de sortie (15),
un opérateur est invité (105) à saisir un avis sur le nombre d'événements non admissibles rapporté à la longueur (72) émis en sortie au moyen d'une unité de saisie (15) et
la limite de nettoyage (6) est fixée automatiquement (109) suivant l'avis saisi.

3. Procédé pour fixer une limite de nettoyage (6) sur une installation de nettoyage électronique (1) pour un échantillon textile allongé (9) tel qu'un fil, dans lequel des valeurs de mesure de plusieurs propriétés de l'échantillon (9) déplacé dans le sens de sa longueur sont captées sur une longueur d'échantillon donnée,
des événements (71, 72) dans l'échantillon (9) sont identifiés à partir des valeurs de mesure,
une représentativité statistique de l'échantillon (9) est déterminée (101) pour chacune des propriétés à partir des événements (71, 72),
une limite de nettoyage (6) constituant un critère d'admissibilité ou de non admissibilité des événements (71, 72) est calculée pour chacune des propriétés, un nombre d'événements non admissibles rapporté à la longueur (72) est calculé pour chacune des propriétés (103) à partir de la représentativité statistique déterminée et de la limite de nettoyage (6),
un nombre total d'événements non admissibles rapporté à la longueur (72) est calculé par totalisation des nombres partiels,
les rapports entre les nombres partiels sont fixés, de préférence automatiquement, de telle façon que le nombre total d'événements non admissibles (72) reste inchangé, la limite de nettoyage (6) est calculée pour chacune des propriétés de façon à obtenir le nombre partiel d'événements non admissibles rapporté à la longueur (72) fixé à partir de la représentativité statistique déterminée et de la limite de nettoyage (6) calculée,
le nombre total d'événements non admissibles rapporté à la longueur (72) est émis sur une unité de sortie (15),
un opérateur est invité (105) à saisir un avis sur le nombre total d'événements non admissibles rapporté à la longueur (72) émis en sortie au moyen d'une unité de saisie (15) et
la limite de nettoyage (6) est fixée automatiquement (109) suivant l'avis saisi.

4. Procédé selon l'une des revendications précédentes, dans lequel l'avis est soit une confirmation (106), auquel cas la limite de nettoyage (6) est laissée inchangée,
soit un ordre de modification (107), auquel cas une nouvelle limite de nettoyage (6) est calculée (108) suivant un ordre de modification et sur la base de la représentativité statistique et un nouveau nombre d'événements non admissibles rapporté à la longueur (72) est calculé à partir de la représentativité statistique déterminée et de la nouvelle limite de nettoyage (6) et émis sur l'unité de sortie (15).

5. Procédé selon la revendication 4, dans lequel l'ordre de modification est soit un ordre d'augmentation du nombre d'événements non admissibles rapporté à la longueur (72), soit un ordre de réduction du nombre d'événements non admissibles rapporté à la longueur (72).

6. Procédé selon la revendication 5, dans lequel l'augmentation ou la réduction du nombre d'événements non admissibles rapporté à la longueur (72) s'effectue selon une valeur d'incrément calculée automatiquement, qui est sensiblement proportionnelle au nombre d'événements non admissibles rapporté à la longueur (72) calculé.

7. Procédé selon l'une des revendications 4 à 6, dans lequel la confirmation (106) ou l'ordre de modification (107) sont effectués par une action unique, par exemple l'actionnement d'un seul bouton.

8. Procédé selon l'une des revendications 4 à 7 dans lequel, après un ordre de modification, l'opérateur est à nouveau invité (105) à saisir un avis sur le nouveau nombre d'événements non admissibles rapporté à la longueur (72) émis en sortie au moyen de l'unité de saisie (15) et la limite de nettoyage (6) est fixée automatiquement (108) suivant l'avis saisi.

9. Procédé selon l'une des revendications précédentes, dans lequel la limite de nettoyage (6) est calculée (102) sur la base de la représentativité statistique de telle manière qu'un nombre d'événements non admissibles rapporté à la longueur (72) par 100 km de longueur testée résultant de la représentativité statistique déterminée et de la limite de nettoyage (6) se situe entre 1 et 200, de préférence entre 10 et 100.

10. Procédé selon l'une des revendications précédentes, dans lequel outre ladite limite de nettoyage (6), une limite de nettoyage des épissures est fixée comme critère pour les épissures admissibles ou non admissibles dans l'échantillon (9) et
ladite limite de nettoyage (6) est fixée de façon à être toujours supérieure à la limite de nettoyage des épissures.

11. Procédé selon l'une des revendications précédentes, dans lequel la limite de nettoyage (6) fixée est automatiquement vérifiée selon au moins un critère prédéterminé et un avertissement est émis si le critère n'est pas satisfait.

12. Procédé selon l'une des revendications précédentes, dans lequel les événements (71, 72) sont définis, d'une part, par les valeurs de mesure (M) et d'autre part par l'étendue (L) des valeurs de mesure dans le sens longitudinal,
un champ d'événements (3) est fourni, qui contient un quadrant ou une partie d'un quadrant d'un système de coordonnées cartésiennes à deux dimensions, dont l'abscisse (31) indique l'étendue (L) des valeurs de mesure dans le sens longitudinal et dont l'ordonnée (32) indique un écart (ΔM) des valeurs de mesure par rapport à une valeur de consigne,
des densités d'événements (71, 72) dans le champ d'événements (3) sont déterminées à partir des valeurs de mesure et de leur étendue (L) dans le sens longitudinal, et
la limite de nettoyage (6) suit pour l'essentiel une densité d'événements constante.

13. Procédé selon l'une des revendications précédentes, dans lequel la limite de nettoyage (6) fixée est réglée sur au moins une tête de mesure (11) de l'installation de nettoyage électronique (1).

14. Procédé selon l'une des revendications précédentes, dans lequel la fixation (102) de la limite de nettoyage (6) s'effectue automatiquement et est déclenchée par une action unique, par exemple l'action sur un seul bouton, un seul clic de souris ou un seul toucher sur un écran tactile.

15. Installation de nettoyage électronique (1) pour un échantillon textile allongé (9) tel qu'un fil, comprenant
au moins une tête de mesure (11) destinée à capter des valeurs de mesure d'au moins une propriété de l'échantillon (9) déplacé dans le sens de sa longueur sur une longueur d'échantillon donnée et
une unité de commande (14) reliée à la tête de mesure (11),
**caractérisée en ce que**
l'unité de commande (14) est conçue pour contrôler le procédé selon l'une des revendications précédentes.
